(19) 

Europäisches
Patentamt
European
Patent Office
Office européen
des brevets

(11) **EP 2 343 379 A1**

(12) **EUROPEAN PATENT APPLICATION**
published in accordance with Art. 153(4) EPC

(43) Date of publication:
**13.07.2011 Bulletin 2011/28**

(21) Application number: **09823336.4**

(22) Date of filing: **29.10.2009**

(51) Int Cl.:
*C12P 19/14* (2006.01)      *B09B 3/00* (2006.01)

(86) International application number:
**PCT/JP2009/005758**

(87) International publication number:
**WO 2010/050223 (06.05.2010 Gazette 2010/18)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(30) Priority: **30.10.2008 JP 2008279367**

(71) Applicant: **Oji Paper Co., Ltd.
Tokyo 104-0061 (JP)**

(72) Inventors:
• **SUGIURA, Jun**
  **Tokyo 104-0061 (JP)**
• **FURUJYO, Atsushi**
  **Tokyo 104-0061 (JP)**

• **CHAO, Yaping**
  **Tokyo 104-0061 (JP)**
• **IGARASHI, Yuko**
  **Tokyo 104-0061 (JP)**
• **IWASAKI, Yuji**
  **Tokyo 104-0061 (JP)**
• **ICHINOMIYA, Masayuki**
  **Tokyo 104-0061 (JP)**
• **SAKAINO, Makoto**
  **Tokyo 104-0061 (JP)**

(74) Representative: **Schmidt, Karsten et al
ZACCO GmbH
Bayerstrasse 83
80335 München (DE)**

(54) **SACCHARIDE PRODUCTION PROCESS AND ETHANOL PRODUCTION PROCESS**

(57)     The present invention provides a pretreatment method which enables the promotion of the enzymatic glycosylation of lignocellulose under relatively mild conditions by using a tree bark as a raw material with less energy. Specifically the present invention provides a step for producing a sugar from a tree bark, which is **characterized by** having the following steps: an alkali treatment step of immersing the tree bark in an alkali compound solution; a refining treatment step of refining the alkali treated tree bark mechanically into fine pieces; and an enzymatic glycosylation step of glycosylating the refined tree bark with an enzyme. The present invention also provides a method of producing an ethanol.

EP 2 343 379 A1

**Description**

BACKGROUND OF THE INVENTION

Field of the Invention

The present invention relates to a method of producing sugar and a method of producing ethanol from a tree bark. Priority is claimed on Japanese Patent Application No. 2008-279367, filed October 30, 2008, the content of which is incorporated herein by reference.

A tree is separated into a xylem and a bark on a boundary of a cambium layer where cell division of the tree is active. Tree bark occupies approximately 10 to 15 % by weight of the total tree weight. Tree bark contains a comparatively low amount of lignin than a xylem and also contains soluble components that make a bark soft. In addition, tree bark is separated into outer bark having dead tissues and inner bark having live tissues.

Outer bark includes mainly a periderm layer or a cork layer. Outer bark protects wood texture from mechanical damage, and also reduces damage from temperature and/or humidity.

An inner bark includes sieve element, parenchyma cell and thick-walled cell. The sieve element functions to transfer liquid and nutrients. The parenchyma cell functions to store nutrients such as starch and the like. The parenchyma cell intercalates between sieve elements of the inner bark. Thick-walled cells function as supporting tissues, the structure of thick-walled cells are layered as seen in the same fashion of annual ring of xylem, and thick-walled cells are separated into bast fiber and sclereid by shapes of thick-walled cells.

Bark tissue mainly includes fine materials such as fibers, corks and parenchyma cells. Fiber of bark is chemically similar to fiber of xylem, and fiber of bark includes cellulose, hemi-cellulose and lignin. Fine materials including cork cells and parenchyma cells contain large amount of extracts such as suberins in a wall of cork cells, and polyphenols in a fine micro material fraction. Accordingly, bark contains a lot of beneficial soluble components compared to xylem. The amount of the beneficial soluble components in bark is 20 to 40% based on the dry weight of the bark, and also bark has an excellent characteristic such that a dry fraction of the bark include fibers that are similar to that of xylem. However, bark is not used for the purpose of lumber. Bark has not beneficially been used as a wood-based biomass since even if commingling of a small amount of bark with pulpification in paper-forming process would decrease quality of the pulp. Therefore, bark is returned to the soil as a fertilizer in plantation along with branches or roots of trees, or peeled and burned at saw mills or chip plants.

In recent years, pine, acacia, eucalyptus and the like are planted as raw materials of pulp for paper-forming. Among those plants, eucalyptus has broadly been planted in worldwide not only for paper-forming but also for greening since eucalyptus has more than 500 types, is capable of being logged for between 7 to 10 years due to its fast growth and grows in dry areas.

On the other hand, beneficial use of a biomass for reducing $CO_2$ emission from fossil fuel from the point of view of preventing global warming has received a lot of attention in recent years. However, in recent years, production of bioethanol from food-based biomass such as corns and the like caused food price hike, and the food hike causes a serious problem for developing countries due to food shortage. Therefore, wood-based biomass such as bioethanol production from a lignocellulose has received a lot of attention since wood-based biomass is not used food source.

Glycosylation which degrades cellulose to monosaccharide such as glucose is an important process when lignocellulose is used.

As a method to produce monosaccharide from lignocellulose, basic three methods are well known such as acid hydrolysis method, hydrolysis method with supercritical water, and enzymic glycosylation method.

Diluted acid method and concentrated acid method are proposed to be used depending on the concentration of acid in the acid hydrolysis method (Patent document 1, Patent document 2). In the diluted acid method, both high temperature and high pressure are required, and acid added will corrode a device. In addition, there are problems such that economically efficient acid recycle method has not been established since it is difficult to separate produced a sugar and acid. Since a concentrated acid method uses relatively low temperature and low pressure, a cheap reaction device can be used and the yield of glucose is high. However, as in the diluted acid method, concentrated acid method is also not capable of separating and recycling economically efficient acid from produced sugar like diluted acid method, resulting in the generation of a large amount of waste acid.

On the other hand, supercritical method which hydrolyzes cellulose with use of subcritical water or supercritical water has been proposed (Patent document 3, Patent document 4). A supercritical method which makes full use of the characteristics of supercritical water is capable of degrading cellulose to oligosaccharide or monosaccharide in short period of time. However, since the degradation is performed under high temperature and high pressure, a device used for the degradation is costly and corrosion of the device may occur due to supercritical water.

In enzymic glycosylation method, lignin and hemi-cellulose in lignocellulose are bound to cellulose, which prevents an enzyme from contacting the cellulose, resulting in low yield of glucose. Accordingly, a pressurized hot water treatment before an enzymic glycosylation, a physical pretreatment by steaming and blasting treatment, or a chemical pretreatment by acid or alkali, are applied to the enzymic glycosylation in order to promote degradation of cellulose by use of an enzyme.

A method of pressurized hot water treatment which treats lignocellulose under high temperature such as 128 to 205 °C and high pressure such as 1 to 2 MPa has been proposed (Patent document 5).

In addition, a method of pressurized hot water treatment which treats lignocellulose under high temperature such as 100 to 500 °C and high pressure such as saturated vapor pressure to 50 MPa has been proposed (Patent document 6).

A method of steaming treatment which treats lignocellulose under high temperature such as 158 to 225 °C and high pressure such as 0.5 to 3 MPa has been proposed (Patent document 7).

In addition, a method of blasting treatment which returns to normal pressure instantly after having maintained lignocellulose under similar condition as steaming treatment has been proposed (Patent document 8).

Each method represented above requires treating lignocellulose under high temperature and high pressure. Therefore, there have been problems such that a costly reaction device is required, and that the input energy to generate high temperature and high pressure is large.

A method of acid treatment which treats lignocellulose with 0.1 to 5 % dilute sulfuric acid at 140 to 230 °C of high temperature, followed by wet crushing the lignocellulose with a freeness machine has been proposed (Patent document 9).

A method of alkali treatment which treats a biomass with 2 to 30 % of calcium hydroxide has been proposed (Patent document 10). Many other alkali treatment methods have been proposed (Patent document 11 to 14).

In each proposed method of the above, glycerol must be crushed between several millimeters to several hundreds micrometers in advance. In addition, since each proposed method of the above requires high temperature and high pressure conditions, there have been problems such that input energy to generate high temperatures and high pressures is large, and the reaction device is costly. In general, as the particle size becomes small, a large amount of energy is required. However, these patent documents do not disclose the amount of energy necessary to crush lignocellulose.

In recent years, varieties of pretreatment of biomass have been proposed. However, if biomass has not been crushed to be less than several millimeters in the pretreatment method, glycosylation efficiency in the glycosylation process which is just after the crushing process would decrease largely. However, if biomass is crushed to less than several millimeters, the energy which is necessary to produce bioethanol will exceed the energy needed to be produced from the biomass.

Therefore, even if bioethanol is produced from biomass, emissions-reduction of $CO_2$ would not be achieved.

[Patent Document 1] Japanese Patent Application Laid-Open No. 2006-75007
[Patent Document 2] Japanese Patent Application Laid-Open No. 2006-246711
[Patent Document 3] Japanese Unexamined Patent Application, First Publication No. Hei 5-31000
[Patent Document 4] Japanese Unexamined Patent Application, First Publication No. Hei 10-327900
[Patent Document 5] Japanese Patent Application Laid-Open No. 2006-136263
[Patent Document 6] Japanese Patent Application Laid-Open No. 2007-20555
[Patent Document 7] Japanese Unexamined Patent Application, First Publication No. Hei 10-117800
[Patent Document 8] Japanese Unexamined Patent Application, First Publication No. Sho 59-204997
[Patent Document 9] Japanese Patent Application Laid-Open No. 2007-124933
[Patent Document 10] Japanese Patent Publication No. 3493026
[Patent Document 11] Japanese Examined Patent Application Publication No. Sho 63-28597
[Patent Document 12] Japanese Unexamined Patent Application, First Publication No. Sho 59-192093
[Patent Document 13] Japanese Unexamined Patent Application, First Publication No. Sho 59-192094
[Patent Document 14] Japanese Patent Application Laid-Open No. 2008-092910

DISCLOSURE OF THE INVENTION

Problems to be Solved by the Invention

The pretreatment method for enzymatic glycosylation of lignocellulose as described above had cost problems such as the pretreatment was carried out under high temperature and high pressure environment, and requiring a large amount of energy for machine crushing.

In addition, the amount of lignin is lower compared to that of the xylem, and the optimized method for making the bark containing large amount of soluble component to the raw material of sugars by enzymatic glycosylation has not been proposed.

More specifically, the present invention provides a pretreatment method which is capable of promoting enzymatic glycosylation of lignocellulose with small amount of energy under a relatively mild condition with use of bark as a raw material.

Means for Solving the Problem

The inventors of the present invention discovered that the amount of lignin is lower compared to that of the xylem. Therefore, the inventors of the present invention focus on tree bark containing large amount of soluble components, by applying the following technical means which is capable of glycosifying tree bark with small amount of energy and producing ethanol.

[1] A method of producing a sugar including: a sugar producing method which produces sugar from tree bark, an alkali treatment step which immerses the tree bark in an alkali compound solution, a refining treatment step which mechanically refines the alkali treated tree bark, and an enzymatic glycosylation step which glycosylates the refined tree bark with an enzyme.

[2] The method of producing the sugar according to [1], wherein the refining treatment is processed by grinding with use of either a device selected from a refiner or a grinder.

[3] A method of producing a sugar, comprising: a refining treatment step which refines a tree bark wherein both of the tree bark and an alkali compound solution are provided to a device selected from a kneader, a biaxially extruder, or a biaxially mixer followed by an alkali treatment and a kneading treatment simultaneously, and an enzymatic glycosylation step which glycosylates the refined tree bark with an enzyme.

[4] The method of producing the sugar according to any one of [1] to [3], wherein the tree bark is ground by a crusher before the alkali treatment, and water retention of the ground tree bark before the alkali treatment is 250 to 2,000 %.

[5] The method of producing the sugar according to any one of [1] to [3], wherein the tree bark is crushed by a uniaxial crusher before the alkali treatment step.

[6] The method of producing the sugar according to any one of [1] to [5], wherein the alkali compound is a calcium hydroxide, separating the tree bark and an alkali solution after the alkali treatment, and returning the calcium hydroxide regenerated from an alkali solution or the alkali solution to the alkali treatment step.

[7] The method of producing the sugar according to any one of [1] to [6], wherein the tree genus of the tree bark belongs to Eucalyptus.

[8] A method of producing ethanol comprising: a fermentation step which ferments the sugar produced by the methods according to any one of [1] to [7].

[9] The method of producing ethanol according to [8], wherein after a fermentation residue generated from the fermentation step is mechanically treated, the fermentation residue is glycosylated and fermented.

[10] The method of producing ethanol, wherein an inorganic content generated from the fermentation residue is calcined to allow the inorganic content to form a calcium oxide when the sugar produced by the method of [7] is fermented, and the calcium oxide is slaked and used in the alkali treatment step.

EFFECT OF THE INVENTION

According to the present invention, bark is enzymatically glycosylated efficiently with less energy, and further providing a method of producing an ethanol. Accordingly, the present invention is capable of producing a bioethanol from tree bark that conventionally has not been used industrially as wood-based resources.

BRIEF DESCRIPTION OF THE DRAWING

Fig. 1 is a flowchart illustrating an example of calcium recycles in a glycosylation step of the present invention.
Fig. 2 is a flowchart illustrating an example of calcium recycles in a glycosylation step of the present invention.
Fig. 3 is a flowchart illustrating an example of calcium recycles in a glycosylation step of the present invention.
Fig. 4 is a flowchart illustrating an example of calcium recycles in a glycosylation step of the present invention.
Fig. 5 is a flowchart illustrating an example of calcium recycles in a glycosylation step of the present invention.
Fig. 6 is a flowchart illustrating an example of a glycosylation and fermentation step of the present invention.
Fig. 7 is a flowchart illustrating an example of a glycosylation and fermentation step of the present invention.

DETAILED DESCRIPTION OF THE INVENTION

The detailed description of the present invention is as follows.
In the method of producing sugars and ethanol of the present invention, bark of woody plants are used as materials. Materials of bark are not particularly limited, however, it is preferable to use the bark belonging to Eucalyptus genus since the bark belonging to Eucalyptus genus have enough thickness and a large amount of sugars (cellulose). Examples of tree genus belonging to Eucalyptus are *Grandis genus, Globulus genus, Nitens genus, Camaldulensis genus, Deglupta genus, Viminalis genus, Urophylla genus, Dunnii genus,* the crossbred and the like.

Eucalyptus bark contains a large amount of calcium oxalate as inorganic compounds. Therefore, the Eucalyptus bark is particularly preferably used as raw materials of bark of the present invention since it is capable of recovering and recycling calcium components in the glycosylation or ethanol fermentation steps of the present invention.

In the present invention, sugars are produced by carrying out the following each steps: an alkali treatment step which immerses bark in a solution of an alkali compound solution, a refining treatment step which refines the tree bark treated with the alkali mechanically, and an enzymatic glycosylation step which glycosylates the refined tree bark with an enzyme.

Materials of bark can be used as they are. As long as the tree bark is cut into several tens $cm^2$ to several $cm^2$ by considering the handling of the tree bark during the transportation, the tree bark can be used as they are for alkali treatment step. If the tree bark is too large, the shape or size of the bark can be reduced to a suitable size by mechanical treatment such as a cutter, a chipper, a crusher, a hammer crusher and the like. The tree bark is preferably refined bark since glycosylation efficiency can be improved in the downstream process of glycosylation step. In the method of the present invention, mechanically treating the bark which that was treated with alkali in the alkali treatment step is capable of refining the bark with less energy. Therefore, it is not necessary to refine raw materials of dried bark before alkali treatment.

The additive amount of alkali compound based on the tree bark in alkali treatment step is not particularly limited as long as the additive amount is enough to soften the bark. The additive amount of alkali compound is preferably used 0.1 parts by mass or more, more preferably used 0.1 to 50 parts by mass, and most preferably 6 to 20 parts by mass based on the 100 parts by mass of the dried bark.

The treatment temperature in the alkali treatment step is not particularly limited as long as the temperature is enough to soften the bark. The treatment temperature is preferably 10 to 300 °C, more preferably 25 to 95 °C, and most preferably 60 to 95 °C. The temperature of less than 10 °C may decrease the effect of an alkali.

In addition, the alkali treatment step is preferably carried out under normal pressure since the alkali treatment step is capable of carrying out with simple equipment and reducing the input energy.

The alkali treating time is not particularly limited as long as the time is enough to soften the tree bark and promote glycosylation of the materials. The treating time is preferably 1 minute to 72 hours, more preferably 3 minutes to 8 hours and most preferably 5 minutes to 1 hour.

In the present invention, tree bark is immersed into an alkali compound solution, and further carries out an alkali treatment by heating the tree bark when needed.

Note that, immersing the tree bark into an alkali compound solution in the present invention may able to be carried out by the following: alkali compound may able to be dissolved in water in advance; tree bark and alkali compounds may be able to be inputted simultaneously into water; tree bark and alkali compound may able to be mixed in advance, followed by further being immersed in water. In any case, tree bark is eventually needed to be immersed in an alkali compound solution.

The concentration of the alkali compound of the alkali compound solution is 0.05 % by mass or more, preferably 0.05 to 10 % by mass, and more preferably 1 to 4 % by mass.

The alkali compound used in the alkali treatment step is not particularly limited as long as the alkali compound is enough to soften the bark. Examples of the alkali compounds are sodium hydroxide, potassium hydroxide, sodium carbonate, sodium hydrogen carbonate, sodium sulfite, ammonia or the mixture of two or more.

Moreover, calcium hydroxide can be used as an alkali compound. Calcium hydroxide is relatively cheaper than the other alkali compounds. In addition, the solubility of calcium hydroxide is lower, therefore, the calcium hydroxide is capable of easily being recovered as a precipitate and reused. Furthermore, although the alkali compound solution is diluted by washing or the like, the collection of calcium content will be easy because calcium carbonate is precipitated when the solution is neutralized with a carbon dioxide.

The solubility of the alkali solution is low when calcium hydroxide is used. Accordingly, the solid content of calcium hydroxide which is not dissolved in the solution has to be included in the solution with the alkali solution simultaneously.

The additive amount of calcium hydroxide is not particularly limited as long as the additive amount of calcium hydroxide is enough to soften the tree bark raw material and promote glycosylation. The additive amount of calcium hydroxide is preferably 0.1 to 50 parts by mass based on the 100 parts by mass of the dried bark. When the additive amount of calcium hydroxide is less 0.1 parts by mass, the glycosylation promotion of tree bark raw material by alkali treatment may not be efficient. When the additive amount of calcium hydroxide exceeds 50 parts by mass, the effect brought by the calcium hydroxide reaches a plateau. Therefore, the additive amount of calcium hydroxide is more preferably 5 to 25 parts by mass.

Additive amount of water is preferably 5 to 20 parts by mass based on one part by mass of the dried tree bark raw material. When the additive amount of water exceeds 20 parts by mass, energy to heat water becomes large, resulting in worse input and output energy balance. When the additive amount of water is less than 5 parts by mass, the contact of the tree bark raw material and calcium hydroxide becomes insufficient, and not enough glycosylation promotion effect may be obtained. Note that, solubility of calcium hydroxide is low such as 1.7 g/L. Accordingly, it is important to letting the calcium hydroxide solution flow to contact the tree bark and the solid calcium hydroxide in the alkali treatment step.

A refining treatment step to mechanically refine a tree bark immersed in an alkali solution is not particularly limited. Examples of refiners which refine a ground bark include a refiner, a grinder or the like.

The grinding treatment of the above means that the alkali treated tree bark raw material is processed with shear force. Examples of grinding devices, a grinder and a refiner that are used for producing pulps can be used. Examples of grinders, a stone-type grinder, a grindstone-type grinder or any one of these can be used.

In addition, examples of refiners, high concentrated refiners that are used for producing mechanical pulps from woods can be used. As types of refiners, single disc refiners that are capable of grinding materials with a stationary plate and a rotating disc; double disc refiners that are capable of grinding materials with two rotating discs that rotate in opposite directions each other; and twin disc refiners that are capable of grinding materials with a stationary plate and rotating discs that are placed on both sides of the stationary plate can be used. Furthermore, conical disc refiners such that the rotating discs are not flat plates but cone-shaped discs can be used.

Furthermore, wet-type media agitating grinding devices can also be used. The devices are capable of grinding the media and fibrous cellulose which is filled in the grinding container by allowing the agitator inserted in the grinding container to rotate at a high speed, causing the media and the fibrous cellulose agitated and generating shear force. For examples, sand grinders are the typical device.

Instead of carrying out the refining treatment step after the alkali treatment, the tree bark raw material is capable of being refined by carrying out the alkali treatment and the kneading treatment simultaneously by mixing the tree bark raw material and the alkali compound solution in a kneader.

The kneading treatment of the above means that the tree bark raw material is refined by physical force while mixing the alkali compound solution. Examples of kneading devices, a kneader, a disperser, an extruder, a mixer or the like can be used. Among these, the kneader which is mainly used for kneading is preferably used, and particularly, a biaxial kneader is more preferably used.

In particular, the bark of Eucalyptus are soft, the bark is easily be refined by a kneader or the like under an alkali compound.

When the refining treatment step is carried out by the kneading treatment, the additive amount of the alkali compound based on the tree bark is not particularly limited. The additive amount of the alkali compound varies in each case, therefore, it is capable of selecting as needed basis.

For examples, when sodium hydroxide is used as the alkali compound, the additive amount of alkali compound is preferably 0.1 parts by mass or more, more preferably 1 parts by mass or more, most preferably 5 parts by mass or more based on the 100 parts by mass of the dried bark. When the additive amount of alkali compound exceeds 30 parts by mass, the effect brought by the alkali compound reaches a plateau.

Accordingly, the additive amount of alkali compound exceeds 30 parts by mass only results in wasting of reagents and wash solutions.

When the refining treatment step is carried out by the kneading treatment, the ratio of the additive amount of the alkali solution based on the tree bark is preferably 1 to 6 when represented by ml based on 1 g of the dried tree bark. When the ratio of the additive amount of the alkali solution based on the tree bark is less than 1, the alkali treatment for the tree bark would not be sufficient, causing inferior glycosylation. In addition, the mechanical energy caused by turning the tree bark into fiber becomes large. When the ratio of the additive amount of the alkali solution exceeds 6, heat energy which is necessary for heating the alkali solution increases, and results in an inefficient refining treatment.

The concentration of the alkali solution which is used for the refining treatment step by the kneading treatment is preferably 5 to 30 %. When the concentration of the alkali solution is less than 5 %, the refining treatment step is not completely done. When the concentration of the alkali solution exceeds 30 %, decomposition of the tree bark is caused.

The treatment temperature of the refining treatment step by the kneading treatment is not particularly limited as long as the treatment temperature is capable of softening the bark, and the temperature is preferably 25 to 300 °C, more preferably 90 to 200 °C. When the temperature is less than 25 °C, the refining treatment step is possibly not completely done. When the temperature exceeds 300 °C, decomposition of the tree bark is caused.

The treating time of the refining treatment step by the kneading treatment is not particularly limited as long as the enough treating time is ensured for softening the bark. The treating time of the refining treatment step by the kneading treatment is preferably 30 seconds to 10 minutes.

The more the bark is treated finely by the kneading treatment, the more efficiency of the following glycosylation increases. However, the more required energy will be necessary for the glycosylation step. Accordingly, it is preferable to use the tree bark fiber in a suitable size range. In particular, average fiber length and average fiber diameter of the tree bark fiber are preferably 2 to 4 mm and 100 to 400 μm, respectively.

The increment of the sugar yield in the following glycosylation step can be achieved by carrying out the refining treatment steps such as grinding or kneading of the above. Since the tree bark is softened by the alkali treatment, the input energy requiring for the refining treatment step has little problem compared to the increment of the sugar yield.

In the present invention, after the tree bark is processed in the alkali treatment step and the refining treatment step, or after the tree bark is processed by the alkali treatment step and the refining treatment step by the kneading treatment, the tree bark is concentrated, washed, or pH adjusted as necessary. After that, the tree bark is processed by the enzymatic glycosylation step with a diastatic enzyme.

In the enzymatic glycosylation treatment step, cellulose component in the refined bark is glycosylated by the diastatic enzyme.

The enzymatic glycosylation treatment step is carried out by applying the same types of enzymes, reaction time, reaction temperature, or the like of the glycosylation treatment step of usual lignocellulosic biomass.

Note that, the enzymatic glycosylation step of the present invention may be capable of fermenting alcohol simultaneously with the glycosylation, or may be glycosylation/fermentation step which is capable of fermenting lactic acid. Hereinafter, the method to ferment a material simultaneously with the glycosylation is called glycosylation/fermentation.

Examples of cellulose degrading enzymes used for glycosylation step include an activated cellobiohydrolase, an activated endoglucanase, and an activated β-glucosidase. These enzymes are so-called cellulase.

In each cellulose degrading enzyme, a suitable amount of the enzyme having specific activity can be added in the glycosylation step. Commercially available cellulase preparation has the each activated cellulase of the above and many of the commercially available cellulase preparation have an activated hemicellulase. Therefore, commercially available cellulase preparation can be used for a cellulose degrading enzyme.

Examples of commercially available cellulase preparation include originated from *Trichoderma, Acremonium, Aspergillus, Phanerochaete, trametes, Humicola,* and *Bacillus.* Examples of the product name of such commercially available cellulase preparation include Cellucine T2 (manufactured by HPI co., ltd), Meicelase (manufactured by Meiji Seika Kaisha, Ltd.), Novozyme 188 (manufactured by Novozymes), MultifectCX10L (manufactured by Genencor), and the like.

The amount used for cellulase preparation based on 100 parts by mass of the raw material solid content is preferably 0.5 to 100 parts by mass and more preferably 1 to 50 parts by mass.

The pH for glycosylation reaction is preferably 4 to 7. The reaction temperature is preferably 30 to 60 °C and more preferably 35 to 50 °C. The reaction step is preferably continuous method; however, a batch method may be used. The glycosylation reaction time varies by enzyme concentration. In the case of a batch method, a reaction time of 0.5 to 72 hours is preferably applied, and more preferably a reaction time of 2 to 48 hours is applied. In case of continuous method, an average detention time is preferably 0.5 to 48 hours and more preferably 1 to 24 hours.

The concentration of the raw material in the glycosylation step is preferably 10 to 30 % by mass. When the concentration is less than 10 % by mass, the concentration of the final product will be too low, and the cost for concentrating the final product will be expensive. In addition, as the concentration becomes high such as exceeds 30 % by mass, stirring of the raw material will be difficult and the productivity will decrease.

The tree bark is preferably sterilized before being processed in an enzymatic glycosylation step. If the tree bark is contaminated with bacteria, the bacteria consume sugars, reducing the yield of the product when the tree bark is glycosylated with an enzyme.

The sterilization step may be carried out by a method to expose the tree bark to the pH ranges which is difficult for the bacterial to grow, with use of acid or alkali; or a method to treat the tree bark under high temperature or a combination of both. The tree bark treated with acid or alkali is preferably used after adjusting the pH of the tree bark near neutral pH, or pH which is suitable for glycosylation and/or glycosylation/fermentation step. When the tree bark is sterilized under high temperature, the tree bark is preferably used after cooling the temperature of the tree bark to room temperature or to a suitable temperature for glycosylation/fermentation step. Using the tree bark after adjusting the temperature or pH is capable of preventing enzyme from losing its activity by being exposed to unsuitable pH ranges or unsuitable temperature ranges.

In the present invention, the tree bark is crushed by a crusher before the alkali treatment, and water retention of the crushed bark before the alkali treatment is preferably 250 to 2,000 %, and more preferably 280 to 400 %. The tree bark is crushed to have a water retention of 250 % or more. Accordingly, alkali is quickly soaked into the crushed tree bark and duration time for alkali treatment can be shortened. If the tree bark is crushed to have its water retention of exceed 2000 %, the energy for crushing the tree bark becomes too large.

In addition, percentage of the water retention of the tree bark of the above is measured as follows.

The crushed tree bark is dried to be a constant mass at 105 ± 3 °C. 100 g of sample is collected from the dried crushed and processed tree bark, and immersed into 1000 g of water for 5 minutes. Five minutes after, the whole amount of the sample is filtered by a screen having 1mm hole (150 mm diameter, 4.2 % hole-area ratio), and measured the amount of the retained water in the crushed and processed tree bark. The percentage of water retention is calculated as follows:

Percentage of water retention = the amount of retained water (g) / 100 g × 100).

In the present invention, crushing the tree bark with use of a uniaxial crusher before the alkali treatment step is preferably carried out for improving the glycosylation efficiency.
A uniaxial crusher has a rotary blade attached to a spinning rotor; a pusher to push a material to the rotary blade; a fixed blade attached to the pusher, and the material is crushed between the rotary blade and the fixed blade.
When tree bark is crushed with a uniaxial crusher, it is capable of crushing the tree bark into fiber with comparatively less input energy. The efficiency of the following glycosylation increases with use of the tree bark fiber. However, the more required energy will be necessary for the glycosylation step. Accordingly, it is preferable to use tree bark fiber in a suitable size range. In particular, the tree bark fiber of 3 mm or more in length is preferably occupy 20 % or more of the entire tree bark fiber. Furthermore, the tree bark fiber of 3 mm or more in length occupying 20 % or more of the entire tree bark fiber as well as the tree bark fiber of 10 mm or less in length occupying 50 % or less is more preferably used. The tree bark fiber of 3 mm or more in length occupying 20 % or more of the entire tree bark fiber as well as the tree bark fiber of 10 mm or more occupying 10 % or less is most preferably used.

In the method of producing sugar of the present invention, it is preferably use calcium hydroxide as an alkali compound, and preferably separates tree bark and the alkali solution after an alkali treatment, and returns the calcium hydroxide regenerated from an alkali solution or the alkali solution to the alkali treatment step.

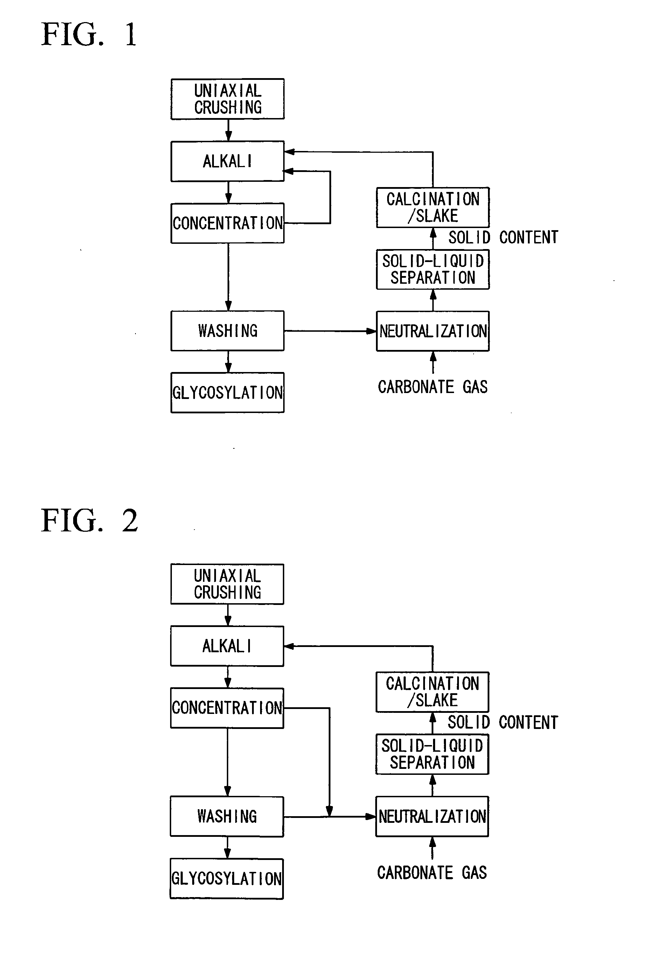

In the method of separating the tree bark and the alkali solution after the alkali treatment, the alkali solution is concentrated by dewatering after the alkali treatment Examples of concentrating the alkali solution include a filtration under normal pressure with use of a filter or the like, a pressure filtration, a suction filtration, or a centrifugal separation. The filtered liquid includes dissolved calcium hydroxide and/or a solidified calcium hydroxide. In the present invention, 20 to 70 % by mass of calcium hydroxide is included in the filtered liquid based on the inputted calcium hydroxide. The filtered liquid is capable of being recycled into the alkali treatment step (Refer to Fig. 1).

By the above cycle, it enables to reduce the amount used of calcium hydroxide and water by recycling the liquid containing calcium hydroxide in the alkali treatment step. Since the calcium hydroxide attached to the solidified dewatered tree bark is sent to the downstream step, it is necessary to add calcium hydroxide when recycling. In addition, the liquid containing calcium hydroxide may be recycled to the alkali treatment step after removing the organic content containing in the liquid by a filtration or the like in the middle of the recycling step when needed.
Furthermore, calcium hydroxide may be recovered from the liquid which was filtered and calcinated, when needed (Refer to Fig. 2).

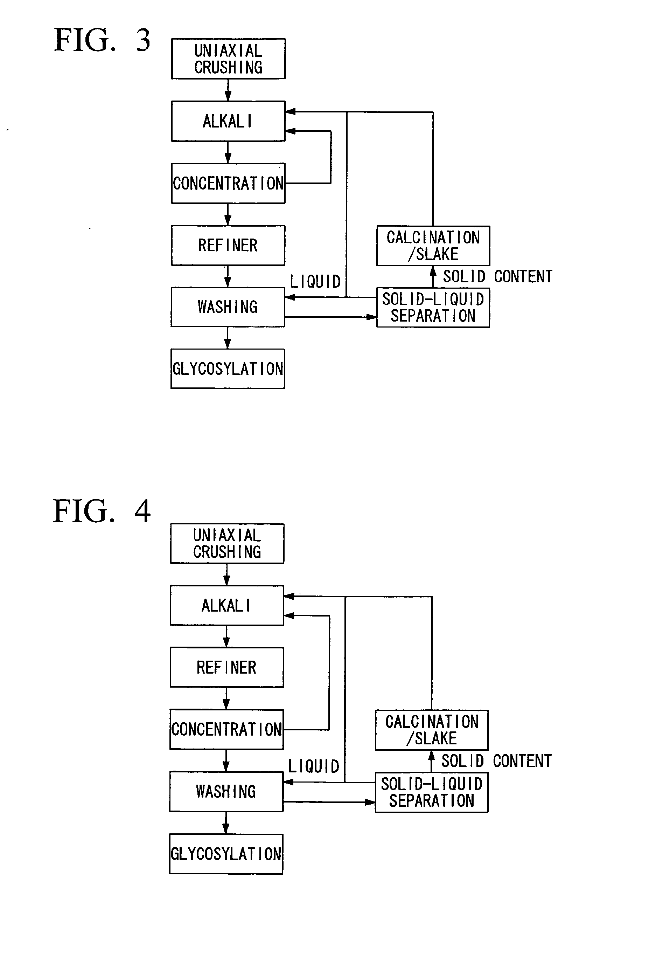

The dewatered tree bark of the above is then treated with a mechanical means as stated above as shown in Fig. 3. In particular, the dewatered tree bark is preferably ground by a refiner or a grinder.

In the grinding treatment, water may be used as needed. In case of the grinding treatment with a refiner, 2 parts by mass or more of water based on 1 part by mass of absolute solid content of the tree bark is preferably used, and more preferably 5 to 20 parts by mass of water based on 1 part by mass of absolute solid content of the tree bark. The grinding treatment may be carried out just after the alkali treatment followed by the concentration treatment as shown in Fig. 4. In such case, alkali is soaked from a newly generated surface of the tree bark fiber by the grinding, improves the effect of the alkali treatment On the other hand, it may reduce efficiency of the concentration treatment if the refined tree bark fiber is generated.

After the tree bark is alkali treated with calcium hydroxide, a washing step is preferably carried out by washing the dewatered tree bark from the alkali solution, or the ground tree bark which produced from the dewatered tree bark from the alkali solution. The washing step is carried out by adding water to the alkali treated material, followed by washing the calcium hydroxide attached to the alkali treated material, or discharging the calcium hydroxide by letting it dissolve in the water. In the washing step, a fall washer, a concentrating washer, a pulp washer or the like may be used. The discharging water generated from this step includes calcium hydroxide or calcium oxalate containing in the tree bark. Since the solubility of these calcium are low, most of the calcium can be recycled as a solid content by carrying out solid-liquid separation. The solid content of the calcium is calcined to form a calcium oxide. The calcium oxide is slaked, and enables it to be reused as a calcium hydroxide.
Before carrying out solid-liquid separation, carbon dioxide is provided to slurry to neutralize the liquid, and recycling of the solid content of the calcium may be promoted (Refer to Fig. 1 and Fig. 2).

In addition, a part or all of the liquid containing calcium hydroxide separated in the solid-liquid separation of the above can be recovered by applying a neutralizing step which neutralizes the liquid by carbon dioxide. The calcium carbonate generated by the neutralization is recovered by precipitating the calcium carbonate in a precipitation bath or the like. The calcium hydroxide is regenerated from the recovered calcium carbonate, and the calcium hydroxide can be reused in the alkali treatment step of the present invention.
The liquid without calcium carbonate can be reused as water for the alkali treatment step and the washing step. Furthermore, the liquid without calcium carbonate can be discarded since its environmental load is small.

The carbon dioxide used for the neutralizing step may be gas or solid, and may be dissolved in liquid.

When the pretreated tree bark of the present invention is glycosylated, and further fermented to produce ethanol, carbon dioxide is generated as a by-product. Therefore, the carbon dioxide is preferably recovered and used for neutralization.

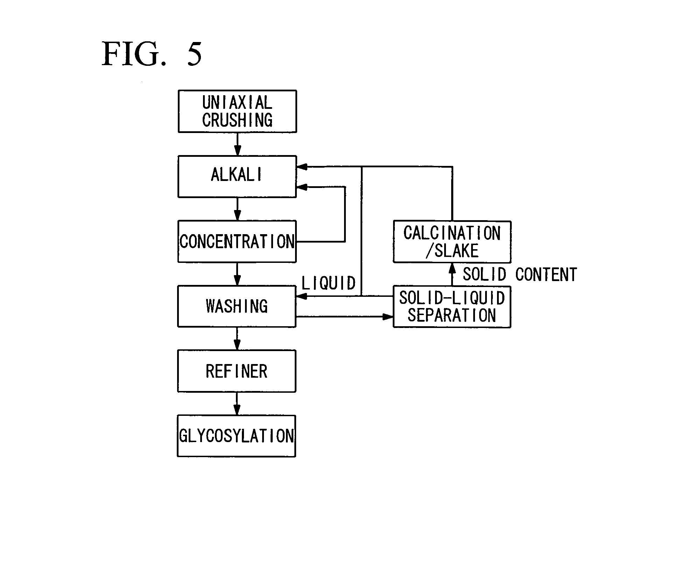

When a grinding step is not carried out after an alkali treatment step by calcium hydroxide, the grinding step may be carried out after a washing step as shown in Fig. 5. In such case, there is an advantage such that a separation of tree bark and liquid in the washing step is easy. However, on the other hand, an alkali concentration in the grinding treatment step may be low.

In the present invention, an ethanol can be produced by carrying out a fermentation step which ferments sugar produced by the sugar production method.

In the fermentation step of the present invention, a fermented product can be produced by fermenting glucose or the like obtained in the previous steps by microorganisms.

The concentration of raw material in the fermentation step is preferably 10 to 30 % by mass. When the concentration is less than 10 % by mass, the concentration of the final product will be too low, and the cost for concentrating the final product will be expensive. In addition, as the concentration becomes high such as exceeds 30 % by mass, stirring of the raw material will be difficult and the productivity will decrease.

In the present invention, an yeast can be used as a microorganism for fermentation, and a culture medium can be used with the microorganism. In particular, *Saccharomyces cerevisiae* or the like can be used.

In addition, a microorganism may be immobilized. Immobilizing microorganism enables to omit a step which runs the microorganism with the liquid and recovers it in the following step. At least immobilizing microorganism enables to reduce a load in the recovering step, and to reduce the risk of losing the microorganism. Furthermore, selecting a microorganism having a coagulation ability enables recovery of the microorganism easily. However, it does not have advantages such as the microorganism immobilization.

A distillation step may be carried out after a fermentation step in the present invention. In the distillation step, a fermented product is separated by distillation in a distillation under reduced pressure device. The fermented product can be separated at low temperature under reduced pressure, therefore it enables to prevent the fermented product from losing enzyme activity. Examples of distillation under reduced pressure devices include a rotary evaporator, a flash evaporator or the like.

The distillation temperature is preferably 25 to 60 °C. When the distillation temperature is less than 25 °C, evaporating the product is time-consuming and the productivity decreases. In addition, when the distillation temperature is more than 60 °C, the enzyme activity is lost due to heat denaturation. Accordingly, economically deteriorates since an additive amount of oxygen which is newly added is increased.

A concentration of the fermented product remained in the residue on the distillation after the distillation is preferably less than 0.1 % by mass. The concentration in such range enables to reduce the amount of fermented product which discharges with the solid in the following solid-liquid separation step, and to improve the yield of ethanol.

As described in the above, glycosylation and fermentation may be carried out in the same step. In such case, applying a cellulose degradative enzyme and a microorganism which is necessary for fermentation enables to carry out glycosylation and fermentation simultaneously.

The pH for glycosylation/fermentation reaction is preferably 4 to 7. The reaction temperature is preferably 25 to 50 °C and more preferably 30 to 40 °C. The reaction step is preferably continuous method; however, batch method may be used. The glycosylation/fermentation reaction time varies by enzyme concentration. In case of batch method, a reaction time of 10 to 240 hours is preferably applied, and more preferably 15 to 160 hours. In case of continuous method, an average detention time is preferably 10 to 150 hours and more preferably 15 to 100 hours.

In the present invention, the step of the both glycosylation and fermentation in succession and a step of both glycosylation and fermentation simultaneously are so-called glycosylation/fermentation (step).

In the above fermentation step, hexose originated from cellulose such as glucose, pentose originated from hemicelluloses such as mannose, galactose or the like are alcoholically fermented. However, some of the pentose remains unreacted pentose. In such case, an enzyme which surely ferments pentose may be added or the unreacted pentose may be treated in another step.

In the ethanol production method of the present invention, after a fermentation residue obtained from the glycosylation/fermentation step is mechanically treated, further glycosylation and fermentation may be carried out.

The mechanical treatment of the fermentation reside means that the residue is ground by arbitrarily mechanical means and allow the residue to be in a suitable form for glycosylation/fermentation. Examples of devices for grinding treatment include the same device used for the grinding treatment (the first grinding treatment) which is the mechanical treatment after the alkali treatment such as a grinder, a refiner or the like.

The fermentation residue after the fermentation is already soft, a use of a refiner is particular preferable. In addition, when the refiner is used in the first grinding treatment, it is preferable to increase the degree of the refining for refining the fermentation residue than the first grinding treatment. When the same refiner is used on both the first grinding treatment and grinding for fermentation residue, it is preferable to use the narrower refiner blade having a clearance at 0.1mm or more for grinding the fermentation residue than that of the first grinding treatment.

An alkali treatment may be carried out before or after the mechanical treatment step of the fermentation residue treatment step.

The same reagents and treatment conditions that are used in the alkali treatment for the tree bark can be used to the alkali treatment of the above step.

From a point of view of the efficiency of the enzymatic glycosylation, adopting the glycosylation/fermentation step simultaneously has big advantages. The mechanical treatment of the fermentation residue in the above case is explained as follows.

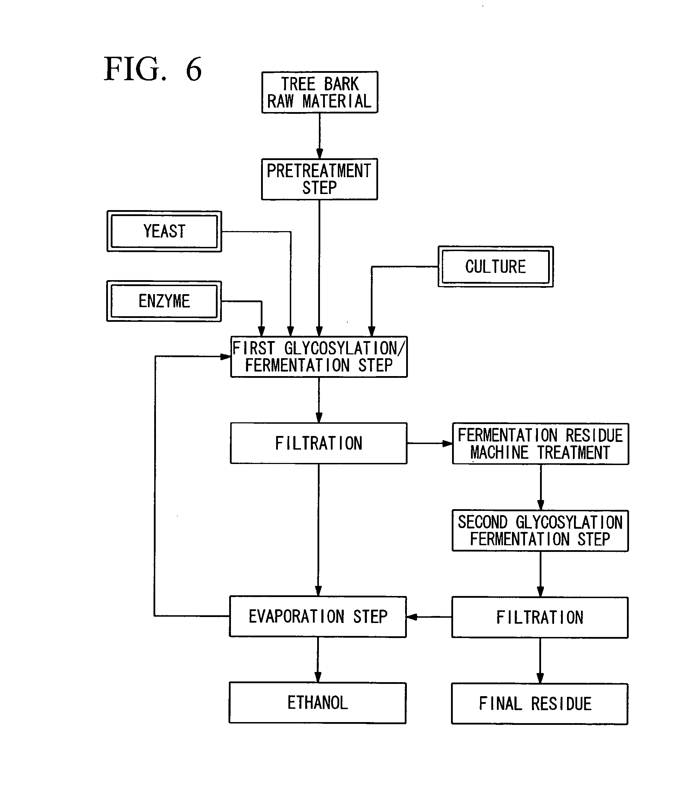

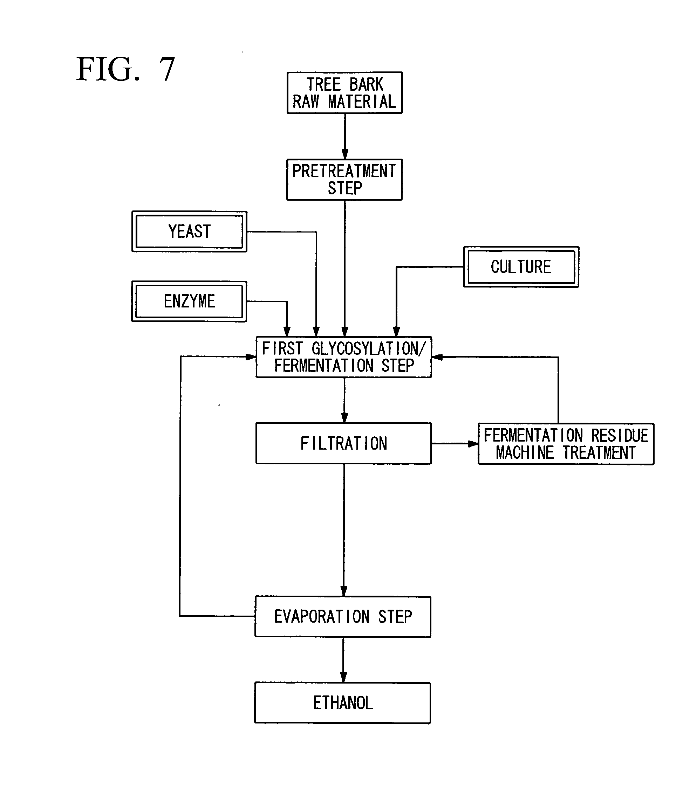

When the fermentation residue obtained from the glycosylation/fermentation step is mechanically treated, further glycosylation and fermentation are carried out, a first embodiment (Refer to Fig. 6) which ferments the residue at another glycosylation/fermentation step (The second glycosylation/fermentation step) form the first glycosylation/fermentation step (The first glycosylation/fermentation step), and a second embodiment (Refer to Fig. 7) which returns the mechanically treated fermentation residue to the first glycosylation/fermentation step are applied.

In the case of the first embodiment, the first glycosylation/fermentation step and the second glycosylation/fermentation step are independent each other, therefore, either a batch method or a continuous method can be used.

In the case of the second embodiment, if the batch method is applied to the first glycosylation/fermentation step, the batch method has to be used for the second glycosylation/fermentation step. The fermentation residue of a first lot of the first glycosylation/fermentation step is treated in the second glycosylation/fermentation step, and the treated residue is mixed to a secondary lot of the first glycosylation/fermentation step. The same of the above are continued afterward. Therefore, the amount of the treated residue provided to a new lot of the first glycosylation/fermentation step is adjusted to be the same amount in every lot, including the residue to be mixed. Furthermore, after several lots are used, it is necessary to discard the residue. This is the same as in the continuous method. It is preferably to continue the treatment in the first glycosylation/fermentation step and the second glycosylation/fermentation step, and it is necessary to discard the residue in arbitrarily timing. The reason to discard the residue is to prevent the glycosylation reaction from inhibition due to accumulation of organic product other than cellulose.

In case of the first embodiment, the fermentation residue which mechanically treated is sent to the second glycosylation/fermentation step. Then, glycosylation/fermentation is carried out in the same manner as the first glycosylation/fermentation step, and solid-liquid separation is carried out by a filtration. The liquid is transported to a evaporation step. The solid is discarded, calcined as the final residue, or provided for a lignin recovery.

Even if glycosylation or fermentation is carried out in any method, a calcium oxide can be obtained by calcinaing the fermentation residue or calcinating the residue which inorganic is removed when calcium hydroxide is used for alkali treatment The calcium oxide is slaked to obtain calcium hydroxide, and the calcium hydroxide can be used for the alkali treatment step. In addition, when a Eucalyp tree bark is used, calcium oxalate in the tree bark remains as a residue. Therefore, a large amount of calcium oxide can be obtained by calcinating the residue.

In particular, it is most reasonable to adopt the glycosylation/fermentation of the above, and carry out the method of calcinating the residue of the second glycosylation/fermentation in the method for grinding treatment of the residue.

EXAMPLES

Hereinafter, a method of producing sugars from tree bark by an alkali treatment step, a refining treatment step, and an enzyme glycosylation step, as well as a method of producing sugars from tree bark by an alkali kneading treatment step and an enzyme glycosylation treatment step are explained in detail in Examples and Comparative Examples.

Note that, % represented in Examples and Comparative Examples in the present invention indicates mass, unless stated otherwise.

<Example 1>

[Alkali treatment]

The bark of *Eucariptus globulus* was cut into approximately 4 cm squares and used as tree bark. 600 g of absolute dry mass of the above tree bark was immersed at 25 °C for 17 hours in an alkali solution of 3000 g in total which contains 60 g of sodium hydroxide and moisture content of the bark. After that, the solid and the liquid were separated by a 40 mesh screen.

[Refining treatment]

The above alkali treated product was ground by a refiner (Kumagai Riki Kogyo Co., Ltd., KRK high concentration disc refiner, the refiner used in Examples and Comparative Examples of the present invention was the same) at a clearance of 1mm and an input speed of 100g/min.

The power consumption of the refiner required for grinding of the above material was calculated by a power accumulator. Note that, the power consumption was calculated by subtracting a power consumption required for idling the refiner (operating the refiner without grinding the tree bark) from the power consumption required for grinding the bark.

[Washing treatment]

A 10 L of pure water was added to the above refined product. After stirring for 1 minute, the washed refined product and the liquid used to wash the refined product were filtered by a 40 mesh screen.

The washed refined product which was obtained by the filtration was applied to a bag made of 420 mesh cloth and then dewatered by a centrifuge.

[Enzyme glycosylation treatment]

The washed refined product which was dewatered of the above was used for an enzyme glycosylation treatment at 30 °C for 20 hours in the following reaction liquid composition.

The amount of sugar produced from the enzyme glycosylation was measured by a Biosenser BF4 (manufactured by Oji Scientific Instruments). The measurement results are shown in Table 1.

(Reaction liquid composition)

5 % tree bark (absolute dry mass of bark)
5 % cellulase (Multifect CX10L, manufactured by Genencor Kyowa K. K)
50 mM acetic acid buffer (pH 4.5)

<Example 2>

A grinding treatment and a glycosylation treatment were carried out in the same manner as Example 1 with the exception of carrying out the alkali treatment by immersing the tree bark in the alkali solution of 95 °C for 90 minutes, and the power consumption required for grinding and the amount of sugar obtained were determined. The evaluation results are shown in Table 1.

<Comparative Example 1>

With use of the xylem of *Eucariptus globules* which was cut into approximately 2 × 4 cm square, an alkali treatment, a grinding treatment and a glycosylation treatment were carried out in the same manner as Example 1, and the power consumption required for grinding and the amount of sugar obtained were determined. The evaluation results are shown in Table 1.

<Comparative Example 2>

With use of the xylem of *Eucariptus globules* which was cut into approximately 2 × 4 cm square, an alkali treatment, a grinding treatment and a glycosylation treatment were carried out in the same manner as Example 2, and the power consumption required for grinding and the amount of sugar obtained were determined. The evaluation results are shown in Table 1.
[Table1]

|  | Power consumption (kWh/t-BD) | Amount of sugar (%) |
|---|---|---|
| Example 1 (tree bark, 25°C) | 35 | 1.50 |
| Comparative Example 1 (Xylem, 25°C) | 772 | 0.56 |
| Example 2 (Tree bark 95°C) | 10 | 2.10 |

(continued)

|  | Power consumption (kWh/t-BD) | Amount of sugar (%) |
|---|---|---|
| Comparative Example 2CXylem, 95°C) | 407 | 0.95 |

According to Table 1, in case of using the bark as a material, the power consumption required for grinding greatly reduced by the alkali treatment compared to the case of using the xylem. In addition, the promotion effect of the enzyme glycosylation by the alkali treatment in case of using bark as a material was larger compared to the case of using the xylem.

<Example 3>

[Alkali treatment]

An alkali treatment was carried out by adding a 2 L of 2.5 % sodium carbonate solution to 1000 g of *Eucariptus globules* bark chip which was passed though a 10 cm screen, and the mixture was autoclaved at 140 °C for 2 minutes.

[Washing treatment]

A 10 L of pure water was added to the above alkali treated product After stirring it for 1 minute, the alkali treated product and the liquid used to wash the alkali treated product were filtered by a 40 mesh screen.
The washed alkali treated product which was obtained by the filtration was applied to a bag made of 420 mesh cloth and then dewatered by a centrifuge.

[Measurement of fiber length and fiber diameter]

A part of the washed product was collected, and the image of the washed product was recorded by a hybrid microscope (SH-4500 manufactured by Hirox Inc.). The image data was binarized and a figure of the washed product was extracted by an image processing/analysis software (IOMate 2007, manufactured by I-spec,Co.,Ltd.), and a boundary length, a vertical feret's diameter, and a horizontal feret's diameter were measured by a figure characteristics measurement. The fiber length was calculated by dividing the boundary length by two. The average fiber diameter was obtained from the smaller value of the vertical/horizontal feret's diameter based on the particles of less than 0.4 degree of circularity. The ratio of each fiber length was indicated in percentage of the number of each fiber based on the number of entire fiber used for the measurement. The average fiber length and the average fiber diameter are shown in Table 2.

[Refining treatment]

The washed and dehydrated product was ground by a refiner (Kumagai Riki Kogyo Co., Ltd., KRK high concentration disc refiner) at a clearance of 1mm and an input speed of 100g/min, and the ground product was obtained.

[Enzyme glycosylation treatment]

After approximately 1 g of the absolute dried ground product was inputted into a 45 ml of 100 mM acetic acid buffer (pH 5.0), 3ml, of cellulase (manufactured by Genencor Kyowa K. K, Multifect CX10L) was added to the solution. The final volume of the solution was adjusted to 50 ml, and an enzymatic glycosylation was carried out at 50 °C for 18 hours. After the reaction, the enzymatically treated product and the enzymatically treated solution was filtered by a 420 mesh screen. The enzymatically treated product was further washed by 100 ml of water, and the water used for washing the enzymatically treated product was mixed to the enzymatically treated liquid. After measuring the volume of the mixture, an amount of sugar containing in the enzymatically treated liquid was measured by a phenol-sulfuric acid method, and the amount of sugar per 1000 g of tree bark solid content was determined. The phenol-sulfuric acid method was referred to as "Method of Determining Reducing Sugar" (Authorized by Sakuzo Fukui, Gakkai Syuppan Center). The amount of sugars obtained above is shown in Table 2.

<Example 4>

An enzymatic glycosylation treatment was carried out in the same manner as Example 3 with the exception of carrying out the alkali treatment by adding 7 L of 2.5 % sodium carbonate solution. In addition, the average fiber length and the average fiber diameter were determined. The results are shown in Table 2.

<Example 5>

[Alkali kneading treatment]

An alkali kneading treatment was carried out by using a *Eucariptus globules* bark chip which was passed though a 10 cm screen and a biaxial kneader (manufactured by Kurimoto ltd., KRC-S2) in the following condition. The air-dried 100 g/min tree bark was inputted into the biaxial kneader, and 2.5 % sodium carbonate solution was simultaneously inputted the biaxial kneader in the speed of 200 ml/min (based on 12.5 g of absolute dry mass of tree bark). The treatment was carried out to be the temperature of the biaxial kneading device to be 140 °C.

[Washing treatment]

A 10 L of pure water was added to the 3000 g of the above alkali kneading treated product After stirring it for 1 minute, the alkali kneading treated product and the liquid used to wash the alkali kneading treated product were filtered by a 40 mesh screen.
The washed alkali kneading treated product which was obtained by the filtration was applied to a bag made of 420 mesh cloth and then dewatered by a centrifuge.
After that, the fiber length and diameter were determined by the method represented in the Example 3, and an enzymatic glycosylation treatment was carried out and the amount of sugars was determined. The results are shown in Table 2.
[Table 2]

|  | Amount of sugar obtained from 1000 g of tree bark raw material (BD) (g) | Amount of alkali solution used (L) | Average fiber length (mm) | Average fiber diameter (μm) |
|---|---|---|---|---|
| Example 3 | 238.4 | 2 | 24.5 | 395 |
| Example 4 | 409.1 | 7 | 23.8 | 367 |
| Example 5 | 411.2 | 2 | 3.8 | 225 |

According to Table 2, it was realized that the amount of sugar obtained based on the same amount of the alkali compound solution was increased when the alkali treatment and the refining treatment of tree bark were carried out simultaneously by the kneader in the alkali kneading treatment step compared to when the alkali treatment carried out by an autoclave and the refining treatment carried out by a refiner are successively performed.
A *Eucariptus globules* bark chip which was passed though a 10 cm screen was crushed by a uniaxial crusher (manufactured by SEIHO KIKO Co., Ltd, SC-15) in the following conditions.
Approximately 25 kg of absolute dried tree bark was inputted into a hopper of the uniaxial crusher, and the uniaxial crusher was operated with a 20 mm round screen attached.
The power consumption of the uniaxial crusher for crushing of the above material was calculated by a power accumulator. Note that, the power consumption was calculated by subtracting a power consumption required for idling the uniaxial crusher (operating the uniaxial crusher without crushing the tree bark) from the power consumption required for crushing the tree bark.
The power consumption of the uniaxial crusher required for crushing the tree bark was 5.3kWh/t-BD.

[Fiber length/Aspect ratio measurement]

The image of the ground product was recorded by a light microscope. The image data was binarized and a figure of the ground product was extracted by an image processing/analysis software (NanoHunter NS2k-Pro, manufactured by NANO System Corporation), and a maximum length and an aspect ratio were determined by a figure characteristics measurement. The ratio of each fiber length was indicated in percentage of the area of each fiber based on the area of entire fiber used for the measurement. In the tree bark which was not fiberized, the maximum length of the fiber direction was used as a fiber length.
The ground product formed large amount of fibers. Ratio of the fiber of 3mm in long diameter and aspect ratio of 100 or more was 0.85 out of 1 when the aspect ratio of the ground product by the image analysis was determined.

[Water retention measurement]

The crushed tree bark is dried to be a constant mass at 105 ± 3 °C. A 100 g of sample is collected from the dried crushed and processed tree bark, and immersed into 1000 g of water for 5 minutes. Five minutes after, the whole amount of the sample is filtered by a screen having 1mm hole (150 mm diameter, 4.2 % hole-area ratio), and determined the amount of the retained water in the crushed and processed tree bark. The percentage of water retention is calculated as follows:

$$\text{Percentage of water retention} = \text{the amount of retained water (g) / 100 g} \times 100).$$

[Alkali treatment]

After approximately 1 kg of the absolute dried crushed product was mixed to 1 L of 12.5 % of calcium hydroxide and adding water to the mixture to be 10 L in total volume, the mixture was heated at 90 °C for 20 minutes, the alkali treated product and the alkali solution were filtered by a 40 mesh screen.
The additive amount of alkali was represented in percentage based on the absolute dried amount of the product to be crushed.

[Washing treatment]

A 10 L of pure water was added to the filtrated alkali treated product of the above. After stirring it for 1 minute, the alkali treated product and the liquid used to wash the alkali treated product were filtered by a 40 mesh screen. The washed alkali treated product which was obtained by the filtration was applied to a bag made of 420 mesh cloth and then dewatered by a centrifuge.

[Refining treatment by grinding]

The above washed alkali treated product was ground by a refiner (Kumagai Riki Kogyo Co., Ltd., KRK high concentration disc refiner) at a clearance of 1mm and input speed of 100g/min, and the ground product was obtained. The power consumption required of the refiner for grinding the tree bark with use of a power accumulator was 50 kWh/t-BD.

[Enzyme glycosylation treatment]

After approximately 1 g of the absolute dried ground product was inputted into a 45 ml of 100 mM acetic acid buffer (pH 5.0), 3ml of cellulase (manufactured by Genencor Kyowa K. K, Multifect CX10L) was added to the solution. The final volume of the solution was adjusted to 50 ml, and an enzymatic glycosylation was carried out at 50 °C for 18 hours. After the reaction, the enzymatically treated product and the enzymatically treated solution was filtered by a 420 mesh screen. The enzymatically treated product was further washed by 100 ml of water, and the water used for washing the enzymatically treated product was mixed to the enzymatically treated liquid. After measuring the volume of the mixture, the amount of sugar containing in the enzymatically treated liquid was measured by a phenol-sulfuric acid method, and the amount of sugar per 1000 g of tree bark solid content was determined. The amounts of sugar obtained above are shown in Table 3.

<Comparative Example 3>

Other than using the xylem of *Eucariptus globules* bark chip which was passed though a 10 cm screen as a raw material, a crushing treatment, an alkali treatment, a washing treatment, a grinding treatment and an enzymatic glycosylation treatment were carried out in the same manner as Example 6.
The power consumption for crushing treatment, the aspect ratio by an image analysis, and the power consumption for grinding were carried out in the same manner as Example 6, and the amount of sugar obtained from 1000 g of tree bark was obtained. The result is shown in Table 3 in comparison with Example 6.

<Example 7>

[Crushing treatment]

A *Eucariptus globules* bark chip which was passed though a 10 cm screen was crushed by a biaxial crusher (manufactured by Kinki Industrial Co., Ltd., PRC-930E) in the following condition.

Approximately 25 kg of absolute dried tree bark was inputted into a hopper of the biaxial crusher, and the biaxial crusher was operated with a 20 mm round screen attached.

The power consumption of the biaxial crusher for crushing of the above material was calculated by a power accumulator.

Note that, the power consumption was calculated by subtracting a power consumption required for idling the biaxial crusher (operating the biaxial crusher without crushing the tree bark) from the power consumption required for crushing the tree bark.

The power consumption of the biaxial crusher required for crushing the tree bark was 13.5 kWh/t-BD.

Hereinafter, the power consumption for crushing treatment, the aspect ratio by an image analysis, and the power consumption for grinding were carried out in the same manner as Example 6, and the amount of sugar obtained from 1000 g of tree bark was obtained. The sugar amount obtained as the result is shown in Table 3.

[Table 3]

| | Consumed power for crushing treatment (kWh/t-BD) | Ratio of the fiber of 3mm in long diameter and aspect ratio of 100 or more | Consumed power for grinding (kWh/t-BD) | Amount of sugar obtained from 1000 g of tree bark raw material (BD) (g) | water retention (%) |
|---|---|---|---|---|---|
| Example 6 | 5.3 | 0.85 | 50 | 473.3 | 280 |
| Example 7 | 13.5 | 0.32 | 252 | 460.0 | 150 |
| Comparative Example 3 | 500 | 0.2 | 1298 | 120 | 120 |

According to Table 3, when xylem was used as a raw material, approximately 12 to 25 times of electric power was required for crushing treatment and grinding treatment compared to a tree bark. In addition, the sugar amount obtained from the 1000 g of raw material fell well below than that of the tree bark.

In addition, the treated product wherein the xylem was crushed in the same condition as the tree bark did not form a fiber since it contained many knobbed products in the treated product The aspect ratio of 100 or more was 0.2.

Hereinafter, a method of producing sugars having a calcium circulation step: a calcium hydroxide solution is used in an alkali treatment step, tree bark and an alkali solution are separated after the alkali treatment step; and the calcium hydroxide regenerated from an alkali solution or the alkali solution is returned to the alkali treatment step, is explained in Examples 8 to 10.

<Example 8>

A *Eucariptus globules* bark chip which was passed though a 10 cm screen was crushed by a uniaxial crusher (manufactured by SEIHO KIKO Co., Ltd, SC-15) in the following condition.

A 217 g of tree bark having 30.8 % by mass of water content (approximately 150 g in absolute dried mass) was inputted into a hopper of the uniaxial crusher, and the uniaxial crusher was operated with use of a φ20 mm round screen.

The power consumption of the uniaxial crusher for crushing of the above material was calculated by a power accumulator.

Note that, the power consumption was calculated by subtracting a power consumption required for idling the uniaxial crusher (operating the uniaxial crusher without crushing the tree bark) from the power consumption required for crushing the tree bark.

The power consumption of the uniaxial crusher required for crushing the tree bark was 6.0 kWh per 1 t of tree bark dry weight

[Alkali treatment (First batch)]

After 18.8 g of Calcium hydroxide powder (based on 12.5 % by mass of absolute dry mass of tree bark) was added to the above crushed tree bark and mixed well. A 1283 g of ion-exchange water was added to the mixture. After mixing it well, the mixture was heated at 90 °C for 40 minutes.

After the alkali treatment, concentration which separates the solid content such as alkali treated product and the liquid was carried out by centrifugal dehydration with use of the 420 mesh screen.

The concentrated alkali treated product (Wet) was 586 g, and the liquid was 73.2 % by mass. Furthermore, the liquid weight was 933 g.

[Enzymatic glycosylation treatment (First batch)]

The alkali treated product of the above was used for the enzymatic glycosylation treatment of the first batch at 50 °C for 20 hours in the following reaction liquid composition ($0^{th}$ cycle).

The result of the obtained sugar yield (g-whole sugar / g-raw material BD) was shown in Table 4.

(Reaction liquid composition)

4 % tree bark (absolute dry mass of bark)
4 % cellulase (Multifect CX10L, manufactured by Genencor Kyowa K. K)
100 mM acetic acid buffer (pH 5.0)

[Circulation step]

In the same manner as above, 13.5 g of Calcium hydroxide powder (based on 9 % by mass of absolute dry mass of tree bark) was added to the 217 g of the crushed tree bark which was crushed by the uniaxial crusher and mixed well. Then, 933 g of the whole amount of the liquid which was separated by centrifugation in the first batch was added to the mixture. Further 362 g of ion-exchange water was added to the mixture, and mixed well. The mixture was heated at 90 °C for 40 minutes, and the alkali treatment of the second batch was obtained. After that, the alkali treated product was inputted to a bag made of 420 mesh cloth and then concentration which separate the solid content such as alkali treated product and the liquid was carried out by centrifugal dehydration. The concentrated alkali treated product (Wet) was 589 g, and the liquid was 72.8 % by mass. Furthermore, the liquid weight was 936 g.

The concentrated alkali treated product was used for the second batch enzymatic treatment in the same manner as the first batch enzymatic treatment. The sugar yield of the second batch enzymatic treatment was shown in Table 4.

<Example 9>

[Washing]

The same alkali treated product of the first batch in Example 8 was produced. A 4570 g of ion-exchange water was added to the alkali treated product After stirring it for 5 minutes, the alkali treated product which is a solid content and the liquid were separated by a centrifugation with use of a 40 mesh screen. The washed product which is solid content (Wet) was 579 g, and the liquid was 73.9 % by mass. The weight of the discharge was 4580 g.

The washed tree bark was used for the enzymatic treatment in the same manner as the first batch enzymatic treatment. The sugar yield of the above was shown in Table 4.

In addition, carbonate gas was mixed to the discharge, and obtained a precipitate. The precipitate was calcined at 850 °C, and obtained calcium oxide. Then, the calcium oxide was slaked to regenerate calcium hydroxide. About 8 g of calcium hydroxide was regenerated.

<Example 10>

The same concentrated alkali treated product of the first batch in Example 8 was produced. A 921 g of ion-exchange water was added to the above product and ground by the refiner (Kumagai Riki Kogyo Co., Ltd.) at a clearance of 0.5 mm.

A 3560 g of ion-exchange water was added to the above ground product. The washing treatment and the enzymatic treatment were carried out in the same manner as the Example 9. The sugar yield was shown in Table 4.

In addition, the discharge generated in the steps of the above was used for regenerating the calcium hydroxide in the same manner as the Example 9. The regenerated calcium hydroxide was about 11 g.

When calcium was collected from the wash solution after the grinding treatment, calcium yield increased. This is assumed that a part of calcium oxalate containing in the tree bark is mixed into the wash solution. Therefore, it is realized that a use efficiency of calcium is further improved.

[Table 4]

| | Example 8 | | Example 9 | Example 10 |
|---|---|---|---|---|
| | First batch | Second batch | | |
| Sugar yield | 0.288 | 0.284 | 0.303 | 0.381 |

According to Example 8, although the usage amount of the calcium hydroxide in the second batch is less than that of the first batch when the discharge generated in the concentration step was used for the alkali treatment step, the same alkali treatment effects could have been achieved. Note that the rate of reduction of calcium hydroxide was about 28 %. The water added newly in the second batch was greatly reduced than the first batch. The exact reason is not know, however, as an effect of the washing treatment, washing treatment may provides an improvement of the sugar yield in the enzymatic treatment. In addition, when calcium is collected from the wash solution, approximately 80 % of the calcium can be collected and used.

Hereinafter, a method of producing ethanol of the present invention is described in Examples 11 to 20.

In Examples 11 to 20, the concentrations of ethanol were measured by a bio censor (manufactured by Oji Scientific Instrument), and amounts of ethanol production were obtained. In addition, the power consumption of the refiner required for pretreatment and (fermentation) residue treatment were calculated by a power accumulator. Note that the power consumption was calculated by subtracting a power consumption required for idling the refiner (operating the refiner without grinding the tree bark) from the power consumption required for grinding the bark.

In addition, yeast such as *Saccharomyces cerevisiae* was used and incubated at 30 °C for 24 hours in the liquid culture having the following composition. Then, the yeast collected by a centrifugation was used in Examples 11 to 20.

(Composition for pre-culture liquid media)

| | |
|---|---|
| Glucose | 30 g/L |
| Polypeptone | 5 g/L |
| Yeast essence | 3 g/L |
| Malt extract | 3 g/L |
| pH | 5.6 |

In addition, a commercially available cellulase manufactured by Genencor Kyowa K. K GC220 (cellobiohydrolase 100 U/mL, β-glucosidase 200 U/mL) was used in Examples 11 to 20.

<Example 11>

*Eucariptus globules* bark chip which was passed though a 10 cm screen was crushed by a uniaxial crusher (manufactured by SEIHO KIKO Co., Ltd, SC-15) having aφ20 mm round screen, was used as the material.

A 500 g of absolute dry mass of the above was mixed to 1.3 L of 10 % sodium carbonate solution. After adding water to the mixture to be 5 L in total volume, the mixture was heated at 100 °C for 30 minutes.

After the alkali treatment, the solid-liquid separation was carried out by a 40 mesh screen and the treated product was ground by a refiner (Kumagai Riki Kogyo Co., Ltd., KRK high concentration disc refiner) at a clearance of 0.5 mm.

A 5 L of pure water was again added to the above ground product. After stirring it for 10 minute, the washed ground product and the liquid used to wash the ground product were filtered by a 40 mesh screen, and the pretreated product was obtained.

The concentration of the pretreated product was adjusted to 8 %, then 3g/L of polypeptone, 2g/L of yeast essence, 2g/L of malt extract were added to the pretreated product The yeast cultured in the 1L of liquid buffer and 200 ml of commercially available cellulase were added to the pretreated product, and glycosylation/fermentation treatment (First glycosylation/fermentation treatment) was carried out at 30 °C for 24 hours. Then, an amount of ethanol obtained by measuring the concentration of ethanol in the glycosylation/fermentation solution was determined. The result is shown in Table 5.

17

<Example 12>

Other than treating the treated product after the alkali treatment with the refiner at a clearance of 0.3 mm, the amount of ethanol production was calculated in the same manner as Example 11. The result is shown in Table 5.

<Example 13>

Other than treating the treated product after the alkali treatment with the refiner at a clearance of 0.2 mm, the amount of ethanol production was calculated in the same manner as Example 11. The result is shown in Table 5.

<Example 14>

Other than treating the treated product after the alkali treatment with the refiner at a clearance of 0.1 mm, the amount of ethanol production was calculated in the same manner as Example 11. The result is shown in Table 5.

<Example 15>

*Eucariptus globules* bark chip which was passed though a 10 cm screen was crushed by a uniaxial crusher (manufactured by SEIHO KIKO Co., Ltd, SC-15) having a φ20 mm round screen, was used as the material.
A 500 g of absolute dry mass of the above was mixed to 1.3 L of 10 % sodium carbonate solution with use of a 10L of stainless bucket After adding water to the mixture to be 5 L in total volume, the mixture was heated at 100 °C for 30 minutes. After the alkali treatment, the solid-liquid separation was carried out by a 40 mesh screen and the treated product was ground by a refiner (Kumagai Riki Kogyo Co., Ltd., KRK high concentration disc refiner) at a clearance of 0.5 mm.
A 5 L of pure water was added to the above ground product After stirring it for 10 minute, the washed ground product and the liquid used to wash the ground product were filtered by a 40 mesh screen, and the pretreated product was obtained (Pretreated product A).
The concentration of the pretreated product A was adjusted to 8 %, then 3g/L of polypeptone, 2g/L of yeast essence, 2g/L of malt extract were added to the pretreated product A. The yeast cultured in the 1L of liquid buffer and 200 ml of commercially available cellulase were added to the pretreated product A, and glycosylation/fermentation treatment (First glycosylation/fermentation treatment) was carried out at 30 °C for 24 hours. Then, an amount of ethanol obtained by measuring the concentration of ethanol in the glycosylation/fermentation solution was obtained.
After the first glycosylation/fermentation treatment, the fermentation residue was obtained by carrying out a solid-liquid separation by a 420 mesh screen. The fermentation residue was refined by the above described refiner at a clearance of 0.3 mm, and the refined fermentation residue was inputted into an empty container. The concentration of the refined fermentation residue was adjusted to 8 % by adding water, and 3g/L of polypeptone, 2g/L of yeast essence, 2g/L of malt extract were added to the refined fermentation residue. The yeast cultured in the 350 mL of liquid buffer and 70 ml of commercially available cellulase were added to the refined fermentation residue, and glycosylation/fermentation treatment (Second glycosylation/fermentation treatment) was carried out at 30 °C for 24 hours. Then, a concentration of ethanol in the glycosylation/fermentation solution was measured, and the total amount of the ethanol obtained in the first glycosylation/fermentation solution and the second glycosylation/fermentation solution was determined. The result is shown in Table 5.

<Example 16>

Other than refining the fermentation residue with the refiner at a clearance of 0.2 mm, the amount of ethanol production was calculated in the same manner as Example 15. The result is shown in Table 5.

<Example 17>

Other than refining the fermentation residue with the refiner at a clearance of 0.1 mm, the amount of ethanol production was calculated in the same manner as Example 15. The result is shown in Table 5.

<Example 18>

In the refining treatment of the fermentation residue, other than not refining the fermentation residue with the refiner at a clearance of 0.3 mm, and the second glycosylation/fermentation was carried out, the amount of ethanol production was calculated in the same manner as Example 15. The result is shown in Table 5.
[Table 5]

| | Refiner clearance (Pretreatment) | Refiner clearance (Residual treatment) | Amount of ethanol produced | Electrical energy (wh/g) | |
|---|---|---|---|---|---|
| | | | | Pre-treatment | Residual treatment |
| Example 11 | 0.5 mm | - | 43.5 g | 224 | - |
| Example 12 | 0.3 mm | - | 46.5 g | 276 | - |
| Example 13 | 0.2 mm | - | 49.0 g | 445 | - |
| Example 14 | 0.1 mm | - | 54.5 g | 816 | - |
| Example 15 | 0.5 mm | 0.3 mm | 59.5 g | 224 | 3 |
| Example 16 | 0.5 mm | 0.2 mm | 60.5 g | 224 | 5 |
| Example 17 | 0.5 mm | 0.1 mm | 61.0 g | 224 | 6 |
| Example 18 | 0.5 mm | untreated | 49.0 g | 224 | 0 |

According to Table 5, the fermentation residue of the tree bark after the enzymatic fermentation step was able to be treated without consuming any electronic power. A total ethanol yield improved by carrying out glycosylation/fermentation repeatedly after the mechanical treatment.

In addition, in the mechanical treatment of the tree bark before the enzymatic glycosylation, the more electrical energy was consumed with less clearance of the refiner, such as at a clearance of 0.1mm and electrical energy of 816 wh/g. However, if the pretreated fiber was refined at a clearance of 0.5 mm and the fermentation residue was refined at a clearance of 0.1 mm, the total electrical energy was able to be reduced to 224 wh/h, and the amount of ethanol production was increased. It is thought that the reduction of the total electric power was achieved by narrowing the fiber using the fermentation treatment and improvement of the ethanol yield was achieved by cellulose exposure by the mechanical treatment of the fermentation residue.

<Example 19>

Experiment was carried out in the same manner as Example 15 until the end of the first glycosylation step. After the first glycosylation/fermentation treatment, the fermentation residue was obtained by carrying out solid-liquid separation with a 420 mesh screen (Fermentation residue C).

On the other hand, once again, the pretreated product A before the first glycosylation step in the same manner as Example 15 was obtained. The pretreated product A and the fermentation residue C were inputted into a reaction container. The concentration of the mixture in the container was adjusted to 8 % by adding water, and 3g/L of polypeptone, 2g/L of yeast essence, 2g/L of malt extract were added to the refined fermentation residue. The yeast cultured in the 1.35 L of liquid buffer and 270 ml of commercially available cellulase were added to the mixture, and the glycosylation/fermentation treatment (First glycosylation/fermentation treatment) was carried out at 30 °C for 24 hours. Then, the concentration of ethanol in the glycosylation/fermentation solution was measured.

The result of Example 19 is shown in Table 6.

<Example 20>

Experiment was carried out in the same manner as Example 15 until the end of the first glycosylation step. After the first glycosylation/fermentation treatment, the fermentation residue was obtained by carrying out solid-liquid separation with a 420 mesh screen. The fermentation residue was refined by the refiner of the above at a clearance of 0.3 mm (Treated residue B).

On the other hand, once again, the pretreated product A before the first glycosylation step in the same manner as Example 15 was obtained. The pretreated product A and the treated residue B were inputted into a reaction container. The concentration of the mixture in the container was adjusted to 8 % by adding water, and 3g/L of polypeptone, 2g/L of yeast essence, 2g/L of malt extract were added to the refined fermentation residue. The yeast cultured in the 1.35 L of liquid buffer and 270 ml of commercially available cellulase were added to the mixture, and a glycosylation/fermentation treatment (First glycosylation/fermentation treatment) was carried out at 30 °C for 24 hours. Then, the concentration of ethanol in the glycosylation/fermentation solution was measured.

The result of Example 20 is shown in Table 6.

[Table 6]

| | Refiner clearance (Pretreatment) | Refiner clearance (Residual treatment) | Amount of ethanol produced | Electrical energy (wh/g) | |
|---|---|---|---|---|---|
| | | | | Pre-treatment | Residual treatment |
| Example 19 | 0.5 mm | Untreated | 46.2 g | 224 | 0 |
| Example 20 | 0.5 mm | 0.3 mm | 56.4 g | 224 | 3 |

<Example 21>

*Eucariptus globules* bark chip which was passed though a 10 cm screen was crushed by a uniaxial crusher (manufactured by SEIHO KIKO Co., Ltd, SC-15) under the following conditions.
A 723 g of tree bark having 30.8 % by mass of water content (approximately 500 g in absolute dried mass) was inputted into a hopper of the uniaxial crusher, and the uniaxial crusher was operated with use of a φ20 mm round screen. The tree bark which was crushed by the uniaxial crusher was collected into a 10 L stainless bucket.

[Alkali treatment]

A 62.4 g of calcium hydroxide powder (based on 12.5 % by mass of absolute dry mass of tree bark) was mixed with the above crushed tree bark, and water was added to the mixture to be 5 L in total volume. After mixing the mixture well, the mixture was heated at 90 °C for 40 minutes.
After the alkali treatment, concentration which separates the solid content such as alkali treated product and the liquid was carried out by centrifugal dehydration with use of the 40 mesh screen.
The concentrated alkali treated product (Wet) was about 1.9 kg, and the liquid was 73.2 % by mass. Furthermore, the liquid weight was 3.1 kg. The liquid was stored as a filtrate (a).

[Grinding treatment]

The concentrated alkali treated product of the above was refined by the refiner (Kumagai Riki Kogyo Co., Ltd., KRK high concentration disc refiner) at a clearance of 0.5 mm.
A 5 L of pure water was added to the above ground product After stirring it for 10 minute, the washed pretreated product (pretreated product (b)) and the liquid used to wash the pretreated product were filtered by a 40 mesh screen. The filtered liquid was stored as a filtrate (c).

[Glycosylation/fermentation treatment]

The pretreated product (b) was inputted into a reaction container, and the concentration of the pretreated product (b) was adjusted to 8 % by adding water to the container. After that, 3g/L of polypeptone, 2g/L of yeast essence, 2g/L of malt extract were added to the pretreated product (b). The yeast cultured in the 1L of liquid buffer and 200 ml of commercially available cellulase were added to the pretreated product (b), and glycosylation/fermentation treatment (First glycosylation/fermentation treatment) was carried out at 30 °C for 24 hours. Then, the amount of ethanol obtained by measuring the concentration of ethanol in the glycosylation/fermentation solution was obtained.
After the first glycosylation/fermentation treatment, the fermentation residue was obtained by carrying out a solid-liquid separation by a 420 mesh screen. The filtered liquid was evaporated in the evaporation step, and ethanol was obtained.
The fermentation residue was refined by the above described refiner at a clearance of 0.3 mm, and the refined fermentation residue was inputted into an empty container. The concentration of the refined fermentation residue was adjusted to 8 % by adding water, and 3g/L of polypeptone, 2g/L of yeast essence, 2g/L of malt extract were added to the refined fermentation residue. The yeast cultured in the 350 mL of liquid buffer and 70 ml of commercially available cellulase were added to the refined fermentation residue, and glycosylation/fermentation treatment (Second glycosylation/fermentation treatment) was carried out at 30 °C for 24 hours. Then, the concentration of ethanol in the glycosylation/fermentation solution was measured, and the total amount of the ethanol obtained in the first glycosylation/fermentation solution and the second glycosylation/fermentation solution was calculated.
After the second glycosylation/fermentation treatment, a final residue was obtained by carrying out solid-liquid separation with use of a 420 mesh screen. The filtered liquid was used in the evaporation step and evaporated.

[Residual and filtrate treatment]

After separating ethanol in the evaporating step, the liquid was returned to the first glycosylation/fermentation step and the solid content and the final residue were calcined to produce calcium oxide.
On the other hand, carbonate gas was mixed to the filtrate (a) and (c), and the produced precipitate and the final residue were calcined. The obtained calcium oxide was approximately 92 g. The amount was far more than the predicted amount from the inputted calcium hydroxide, and it is assumed that most of the calcium oxalate of the tree bark was recovered as the calcium hydroxide.

INDUSTRIAL AVAILABILITY

The present invention provides a method of carrying out an enzymatic glycosylation of a tree bark with less energy and ethanol production. The present invention also provides a method of producing bioethanol from a tree bark which had not been conventionally used in industries as wood-based resources.

**Claims**

1. A method of producing a sugar comprising:

    a sugar producing method which produces sugar from a tree bark,
    an alkali treatment step which immerses the tree bark in an alkali compound solution,
    a refining treatment step which mechanically refines the alkali treated tree bark, and
    an enzymatic glycosylation step which glycosylates the refined tree bark with an enzyme.

2. The method of producing the sugar according to claim 1, wherein the refining treatment is processed by a grinding with use of a device selected from a refiner or a grinder.

3. A method of producing a sugar, comprising:

    a refining treatment step which refines a tree bark wherein both of the tree bark and an alkali compound solution are provided to a device selected from a kneader, a biaxially extruder, or a biaxially mixer followed by an alkali treatment and a kneading treatment simultaneously, and
    an enzymatic glycosylation step which glycosylates the refined tree bark with an enzyme.

4. The method of producing the sugar according to any one of claims 1 to 3, wherein the tree bark is ground by a crusher before the alkali treatment, and water retention of the ground tree bark before the alkali treatment is 250 to 2,000 %.

5. The method of producing the sugar according to any one of claims 1 to 3, wherein the tree bark is crushed by a uniaxial crusher before the alkali treatment step.

6. The method of producing the sugar according to any one of claims 1 to 5, wherein the alkali compound is a calcium hydroxide, separating the tree bark and an alkali solution after the alkali treatment, and returning the calcium hydroxide regenerated from an alkali solution or the alkali solution to the alkali treatment step.

7. The method of producing the sugar according to any one of claims 1 to 6, wherein a tree genus of the tree bark belongs to Eucalyptus.

8. A method of producing ethanol comprising:

    a fermentation step which ferments the sugar produced by the methods according to any one of claims 1 to 7.

9. The method of producing ethanol according to claim 8, wherein after a fermentation residue generated from the fermentation step is mechanically treated, the fermentation residue is glycosylated and fermented.

10. The method of producing ethanol, wherein an inorganic content generated from the fermentation residue is calcined to allow the inorganic content to form a calcium oxide when the sugar produced by the method of claim 7 is fermented, and the calcium oxide is slaked and used in the alkali treatment step.

FIG. 1

```
      ┌──────────────┐
      │  UNIAXIAL    │
      │  CRUSHING    │
      └──────┬───────┘
             │
             ▼
      ┌──────────────┐◄────────────────────────────┐
      │    ALKALI    │◄──────────────┐              │
      └──────┬───────┘               │      ┌───────┴────────┐
             │                       │      │  CALCINATION   │
             ▼                       │      │    /SLAKE      │
      ┌──────────────┐               │      └───────▲────────┘
      │ CONCENTRATION │──────────────┘          SOLID CONTENT
      └──────┬───────┘                      ┌────────────────┐
             │                              │  SOLID-LIQUID  │
             │                              │   SEPARATION   │
             │                              └───────▲────────┘
             ▼                                      │
      ┌──────────────┐              ┌───────────────┴────┐
      │   WASHING    │─────────────►│   NEUTRALIZATION   │
      └──────┬───────┘              └───────────▲────────┘
             │                                  │
             ▼                             CARBONATE GAS
      ┌──────────────┐
      │ GLYCOSYLATION │
      └──────────────┘
```

FIG. 2

```
      ┌──────────────┐
      │  UNIAXIAL    │
      │  CRUSHING    │
      └──────┬───────┘
             │
             ▼
      ┌──────────────┐◄────────────────────────────┐
      │    ALKALI    │                              │
      └──────┬───────┘                      ┌───────┴────────┐
             │                              │  CALCINATION   │
             ▼                              │    /SLAKE      │
      ┌──────────────┐                      └───────▲────────┘
      │ CONCENTRATION │─────────┐               SOLID CONTENT
      └──────┬───────┘          │          ┌────────────────┐
             │                  │          │  SOLID-LIQUID  │
             │                  │          │   SEPARATION   │
             │                  │          └───────▲────────┘
             ▼                  ▼                   │
      ┌──────────────┐   ┌───────────────┐  ┌───────┴────────┐
      │   WASHING    │──►│ NEUTRALIZATION │  │                │
      └──────┬───────┘   └───────▲───────┘
             │                   │
             ▼              CARBONATE GAS
      ┌──────────────┐
      │ GLYCOSYLATION │
      └──────────────┘
```

23

FIG. 3

```
      ┌──────────────┐
      │  UNIAXIAL    │
      │  CRUSHING    │
      └──────────────┘
             │
             ▼
      ┌──────────────┐◄──────────────────┐
      │    ALKALI    │◄──────────────┐   │
      └──────────────┘◄──────┐       │   │
             │               │       │   │
             ▼               │       │   │
      ┌──────────────┐       │       │   │
      │CONCENTRATION │───────┘       │   │
      └──────────────┘               │   │
             │                       │   │
             ▼                       │   │
      ┌──────────────┐        ┌──────────────┐
      │   REFINER    │        │  CALCINATION │
      └──────────────┘        │    /SLAKE    │
             │                └──────────────┘
             │        LIQUID     ▲  SOLID CONTENT
             ▼                   │
      ┌──────────────┐    ┌──────────────┐
      │   WASHING    │◄───│ SOLID-LIQUID │
      │              │───►│  SEPARATION  │
      └──────────────┘    └──────────────┘
             │
             ▼
      ┌──────────────┐
      │ GLYCOSYLATION│
      └──────────────┘
```

FIG. 4

```
      ┌──────────────┐
      │  UNIAXIAL    │
      │  CRUSHING    │
      └──────────────┘
             │
             ▼
      ┌──────────────┐◄──────────────────┐
      │    ALKALI    │◄──────────────┐   │
      └──────────────┘◄──────┐       │   │
             │               │       │   │
             ▼               │       │   │
      ┌──────────────┐       │       │   │
      │   REFINER    │       │       │   │
      └──────────────┘       │       │   │
             │               │       │   │
             ▼               │       │   │
      ┌──────────────┐       │       │   │
      │CONCENTRATION │───────┘       │   │
      └──────────────┘               │   │
             │                       │   │
             │        LIQUID  ┌──────────────┐
             ▼                │  CALCINATION │
      ┌──────────────┐        │    /SLAKE    │
      │   WASHING    │        └──────────────┘
      │              │           ▲  SOLID CONTENT
      └──────────────┘           │
             │        ┌──────────────┐
             │◄───────│ SOLID-LIQUID │
             │───────►│  SEPARATION  │
             ▼        └──────────────┘
      ┌──────────────┐
      │ GLYCOSYLATION│
      └──────────────┘
```

FIG. 5

FIG. 6

FIG. 7

```
                    ┌──────────────┐
                    │ TREE BARK    │
                    │ RAW MATERIAL │
                    └──────┬───────┘
                           ▼
                    ┌──────────────┐
                    │ PRETREATMENT │
                    │    STEP      │
                    └──────┬───────┘
  ┌──────────┐            │
  │  YEAST   │───┐        │
  └──────────┘   │        │        ┌──────────┐
                 │        │        │ CULTURE  │
  ┌──────────┐   │        │        └────┬─────┘
  │  ENZYME  │─┐ │        │             │
  └──────────┘ │ │        │             │
               ▼ ▼        ▼             ▼
        ┌─────────────────────────────────┐
   ┌───►│ FIRST GLYCOSYLATION/            │◄──────┐
   │    │ FERMENTATION STEP               │       │
   │    └────────────────┬────────────────┘       │
   │                     ▼                         │
   │            ┌──────────────┐    ┌────────────────────────┐
   │            │  FILTRATION  │───►│ FERMENTATION RESIDUE   │
   │            └──────┬───────┘    │ MACHINE TREATMENT      │
   │                   ▼            └────────────────────────┘
   │            ┌──────────────────┐
   └────────────│ EVAPORATION STEP │
                └──────┬───────────┘
                       ▼
                ┌──────────────┐
                │   ETHANOL    │
                └──────────────┘
```

27

<table>
<tr><td colspan="2" align="center">**INTERNATIONAL SEARCH REPORT**</td><td colspan="2">International application No.<br>PCT/JP2009/005758</td></tr>
</table>

A. CLASSIFICATION OF SUBJECT MATTER
*C12P19/14*(2006.01)i, *B09B3/00*(2006.01)i

According to International Patent Classification (IPC) or to both national classification and IPC

B. FIELDS SEARCHED

Minimum documentation searched (classification system followed by classification symbols)
C12P19/14, B09B3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
JSTPlus/JMEDPlus/JST7580(JDreamII), BIOSIS(STN), CAplus(STN), WPI

C. DOCUMENTS CONSIDERED TO BE RELEVANT

| Category* | Citation of document, with indication, where appropriate, of the relevant passages | Relevant to claim No. |
|---|---|---|
| Y | WO 2008/090707 A1 (Japan International Research Center for Agricultural Sciences), 31 July 2008 (31.07.2008), & JP 2008-178355 A | 1-10 |
| Y/A | WO 2008/123419 A1 (National Institute of Advanced Industrial Science and Technology), 16 October 2008 (16.10.2008), & JP 2008-274247 A | 1-2,4-10/3 |
| Y/A | JP 59-192093 A (Shinnenryoyu Kaihatsu Gijutsu Kenkyu Kumiai), 31 October 1984 (31.10.1984), & US 4642287 A          & AU 2675484 A & BR 8401702 A | 3-10/1-2 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>08 December, 2009 (08.12.09) | Date of mailing of the international search report<br>15 December, 2009 (15.12.09) |
|---|---|
| Name and mailing address of the ISA/<br>Japanese Patent Office | Authorized officer |
| Facsimile No. | Telephone No. |

Form PCT/ISA/210 (second sheet) (April 2007)

28

**INTERNATIONAL SEARCH REPORT**

International application No.

PCT/JP2009/005758

C (Continuation).   DOCUMENTS CONSIDERED TO BE RELEVANT

| Category* | Citation of document, with indication, where appropriate, of the relevant passages | Relevant to claim No. |
|---|---|---|
| Y/A | JP 8-503126 A  (The Texas A & M University System), 09 April 1996 (09.04.1996), & US 5865898 A        & EP 654096 A & WO 1994/003646 A1      & DE 69333708 D & CA 2141794 A | 6-7,9-10/ 1-5,8 |
| Y/A | JP 63-137692 A  (Kobe Steel, Ltd.), 09 June 1988 (09.06.1988), (Family: none) | 9/1-8,10 |
| P,Y | JP 2009-125050 A  (JFE Engineering Corp.), 11 June 2009 (11.06.2009), & WO 2009/069323 A1 | 1-10 |
| P,A | Yasuyuki MATSUSHITA et al., "Juhi no Koso Toka ni Okeru Shinki Maeshori Gijutsu no Kaihatsu", Dai 53 Kai Proceedings of the Lignin Symposium, 30 October 2008 (30.10.2008), pages 96 to 97 | 1-10 |
| P,A | Keiji TAKABE et al., "Yukari Juhi no Lignin Bunpu to Koso Tokasei", Dai 53 Kai Proceedings of the Lignin Symposium, 30 October 2008 (30.10.2008), pages 98 to 101 | 1-10 |
| A | JP 2007-124933 A  (Tsukishima Kikai Co., Ltd.), 24 May 2007 (24.05.2007), (Family: none) | 1-10 |

Form PCT/ISA/210 (continuation of second sheet) (April 2007)

**REFERENCES CITED IN THE DESCRIPTION**

**Patent documents cited in the description**

- JP 2008279367 A
- JP 2006075007 A
- JP 2006246711 A
- JP HEI531000 B
- JP HEI10327900 B
- JP 2006136263 A
- JP 2007020555 A
- JP HEI10117800 B

- JP SHO59204997 B
- JP 2007124933 A
- JP 3493026 B
- JP SHO6328597 B
- JP SHO59192093 B
- JP SHO59192094 B
- JP 2008092910 A